# EUROPEAN PATENT APPLICATION

(11) **EP 3 868 895 A1**
(43) Date of publication of application: **25.08.2021**
(21) Application number: 20382121.0
(22) Date of filing: 20.02.2020
(51) Int. Cl.: C12Q 1/6883

(54) **IN VITRO METHOD FOR THE DIAGNOSIS AND/OR PROGNOSIS OF CALCIFIC AORTIC VALVE DISEASE OR SUBCLINICAL AORTIC-VALVE CALCIFICATION**

(71) Applicant: Centro Nacional de Investigaciones Cardiovasculares Carlos III (F.S.P.), 28029 Madrid (ES)
(72) Inventor: MacGrogan, Donal, 28029 Madrid (ES); Sánchez Cabo, Fátima, 28029 Madrid (ES); Fernández Friera, Leticia, 28029 Madrid (ES); de la Pompa, José Luis, 28029 Madrid (ES)
(74) Representative: Hoffmann Eitle

(57) **Abstract**

*In vitro* method for the diagnosis and/or prognosis of calcific aortic valve disease or subclinical aortic-valve calcification. The present invention refers to an in vitro method and kit for the diagnosis of calcific aortic valve disease (CAVD) or subclinical aortic-valve calcification, and/or for the differential diagnosis between CAVD and subclinical aortic-valve calcification.

## Description

### FIELD OF THE INVENTION

The present invention pertains to the medical field. More specifically, the present invention refers to an *in vitro* method for the diagnosis and/or prognosis of calcific aortic valve disease (CAVD) or subclinical aortic-valve calcification, and/or for the differential diagnosis between CAVD and subclinical aortic-valve calcification. The present invention also comprises kits adapted for the diagnosis of CAVD or subclinical aortic-valve calcification, and/or for the differential diagnosis between CAVD and subclinical aortic-valve calcification.

### PRIOR ART

CAVD is caused by calcium build up and stiffening of the aortic valve leaflet. CAVD, especially when there is severe calcification, may be associated with haemodynamic obstruction, leading to different degrees of aortic stenosis (AS). Untreated severe AS leads to heart failure, with valve replacement remaining the only available treatment. Severe AS is preceded by aortic sclerosis, a subclinical form of CAVD that develops over many years without haemodynamic consequence. The prevalence of aortic valve disorders increases with age, with 25% of individuals >65 years with aortic sclerosis progressing to AS at a rate of 2% per year. Other than age, the principal CAVD risk factor for AS is a congenitally malformed bicuspid aortic valve (BAV), which is present in 0.5-2% of the general population. AS patients with a BAV are a decade younger at diagnosis than those with normal tricuspid aortic valve (TAV) morphology. Premature leaflet thickening and calcification in patients with BAV may involve abnormal shear stress haemodynamics.

CAVD is an active process that shares mechanistic features with atherosclerosis. The current accepted model implicates initial lipid oxidation, inflammatory infiltration by T-lymphocytes and macrophages, cytokine and pro-fibrotic signalling, and altered extracellular matrix (ECM) secretion, leading to ectopic bone formation and tissue mineralisation. The factors predicting transition from the "at risk" status to aortic sclerosis and subsequently to severe AS, are not known. Transcriptional profiling of CAVD has helped to identify hundreds of genes differentially expressed between calcified and normal valves. Multi-omics approaches combining transcriptomics and proteomics have provided a high-resolution expression atlas and established potential drivers of disease progression.

Despite this progress, thus far no medical therapy has been proven to be effective in preventing progression to severe disease, with randomised clinical trials demonstrating the lack of effect of statins on disease progression. However, it has been suggested that statin treatment would have to begin at an earlier disease stage to have a beneficial effect. Current imaging modalities, especially echocardiography, remain the gold standard for diagnosis of valve disease but are mainly performed when symptoms appear. Non-contrast computed tomography may have significant predictive value in both asymptomatic and symptomatic patients but is a source of radiation exposure and it may not be available in centres with limited resources. Biomarkers may complement imaging modalities to detect early changes, especially early calcification. The ideal biomarker would predict disease as well as improve prognosis, subclinical disease management, and risk stratification and thus reduce disease-associated morbidity and mortality.

The aortic valve develops from rudimentary endocardial cushions that are subsequently refined into thin leaflets well into postnatal life. This process is dependent on complex cell-ECM interactions and is modulated by haemodynamics, and its spatial-temporal dysregulation results in malformations and maladaptive remodelling. Several lines of evidence indicate that valve disease at any age is accompanied by some underlying structural defect, suggesting that latent CAVD has a developmental origin. For example, conserved mechanisms common to valvulogenesis and bone development may play a significant role in promoting or accompanying CAVD progression. However, only end-stage CAVD has been described at the molecular level, whereas the molecular underpinnings of subclinical disease are poorly understood, due in part to difficulties in accessing biological material from early lesions.

Consequently, there is an unmet medical need of finding reliable methods for the diagnosis of CAVD and/or subclinical aortic-valve calcification. The identification of early CAVD biomarkers is critical for optimizing therapy decisions and patient follow-up and deepen our understanding of normal and disease states. Thus, the present invention is focused on solving that problem and specific biomarkers and signatures are herein provided which can be efficiently used for the diagnosis of CAVD and/or subclinical aortic-valve calcification.

### DESCRIPTION OF THE INVENTION

### Brief description of the invention

The inventors of the present invention have performed an RNA-seq analysis of human foetal and adult control and calcified bicuspid (cBAV) and tricuspid valves (cTAV) and combined this with a meta-analysis of a previous study. K-means clustering was used to identify dynamically regulated processes taking place simultaneously at crucial stages of human valve development and in diseased valves. The prevailing gene signatures were associated with immune-metabolic processes and heretofore uncharacterized pathways linked to cellular stress and ageing. A search for genes shared between the gene expression datasets and datasets generated from asymptomatic subjects from the Progression of Early Subclinical Atherosclerosis (PESA) cohort with aortic valve calcification and without functional stenosis identified marker genes in peripheral blood of asymptomatic individuals. When combined in multiplex quantitative RT-PCR assays, these marker genes showed high sensitivity and specificity for predicting CAVD.

Thus, by comparing the CAVD transcriptomes and whole blood from datasets of the subclinical PESA study population, the inventors of the present invention have identified six differentially expressed genes, four of which were found to predict subclinical and overt valve disease. Therefore *ASCC3, ITK, CD28,* and *LINS1* were specific to aortic valve calcification, whereas *PAG1* and *NLRC6* were also associated with coronary artery calcification, suggesting that valve and vascular calcification proceed by shared and separate mechanisms. It is noteworthy that five of six genes belong to T cell activation and NF-kB pathways. This gene signature could be used in combination with high-resolution imaging for the early diagnosis of valve calcification.

Such as it is explained in the Examples of the present invention, a model has been built using the expression measures of the four genes specifically associated with aortic valve calcification: ASCC3, ITK, CD28 and LINS1. ROC curves were built to represent the predictive value of the model to classify:
- CAVD patients (WB-CAVD) vs healthy control subjects (individuals without calcification) (PESA-CTL). The AUC value for the first cohort was 0.91 and the AUC value for the validation cohort was 0.88. These results give rise to the development of an *in vitro* method for the diagnosis of CAVD.
- Subjects with subclinical aortic-valve calcification (PESA-SD individuals) vs healthy control subjects (individuals without calcification) (PESA-CTL). The AUC value for both the first cohort and the validation cohort was 0.71. These results give rise to the development of an *in vitro* method for the diagnosis of subclinical aortic-valve calcification.
- CAVD patients (WB-CAVD) vs control patients with subclinical aortic-valve calcification (PESA-SD individuals). The AUC value for the first cohort was 0.97 and the AUC value for the validation cohort was 0.90. These results give rise to the development of an *in vitro* method for the differential diagnosis between CAVD and subclinical aortic-valve calcification.

Thus, the model designed in the present invention identifies a gene expression signature in blood that is highly predictive of valve disease and of aortic valve calcification levels in otherwise healthy individuals.

Consequently, the first embodiment of the present invention refers to an *in vitro* method for the diagnosis and/or the prognosis of CAVD or subclinical aortic-valve calcification which comprises: a) Measuring the expression level of a combination of genes comprising at least: [ASCC3, ITK, CD28 and LINS1] in a biological sample obtained from the patient, b) processing the expression level values in order to obtain a risk score and c) wherein if a deviation or variation of the risk score value is identified, as compared with a reference expression level measured in healthy control subjects, this is an indication that the subject is suffering from CAVD or subclinical aortic-valve calcification.

The second embodiment of the present invention refers to the *in vitro* use of a combination of genes comprising at least: [ASCC3, ITK, CD28 and LINS1] for the diagnosis and/or prognosis of CAVD or subclinical aortic-valve calcification.

In a preferred embodiment the method of the invention refers to an *in vitro* method for the differential diagnosis between CAVD and subclinical aortic-valve calcification which comprises: a) Measuring the expression level of a combination of genes comprising at least: [ASCC3, ITK, CD28 and LINS1] in a biological sample obtained from the patient, b) processing the expression level values in order to obtain a risk score and c) wherein if a deviation or variation of the risk score value is identified, as compared with the reference expression level measured in subclinical aortic-valve calcification control patients, this is an indication that the subject is suffering from CAVD.

In a preferred embodiment, the biological sample is selected from: whole blood, serum or plasma.

In a preferred embodiment, the diagnosis is confirmed by imaging techniques, preferably ultrasound scan.

The third embodiment of the present invention refers to a kit adapted for the diagnosis of CAVD, for the diagnosis of subclinical aortic-valve calcification, or for the differential diagnosis between CAVD and subclinical aortic-valve calcification which comprises reagents for the determination of the expression level of a combination of genes comprising at least: [ASCC3, ITK, CD28 and LINS1].

In a preferred embodiment, the deviation or variation of the level of expression of the genes refers to an overexpression as compared with the level of expression measured in control subjects.

Thus, in a preferred embodiment, the patients can be stratified and monitored according to the expression level of the combination of genes comprising at least: [ASCC3, ITK, CD28 and LINS1], in order to identify a subset of patients that will progress or very likely progress towards CAVD.

The fourth embodiment of the present invention refers to a method for treating CAVD and/or subclinical aortic-valve calcification which comprises an initial (or previous) step directed to the diagnosis of the disease by using the methods described in the present invention. In other words, once the patient is diagnosed with CAVD and/or subclinical aortic-valve calcification, following the method described in the present invention, these patients can be treated with any of the treatments available in the prior art, for example surgical aortic valve replacement with a mechanical or bioprosthetic valve, or transcatheter aortic valve implantation. On the other hand, antibiotics may be used in case of infections and vasodilators for acute decompensation.

Thus, the present invention has a clear utility at clinical level because, by way of example, the combination of genes of the present invention could be used in emergency services to rapidly rule out important aortic diseases, before the patient is examined by the cardiologist using imaging techniques.

In addition, it could be interesting to use the combination of genes of the present invention to monitor patients in primary care services, where there are generally no cardiologists or imaging techniques to carry out an accurate diagnosis. In this way, the presence of calcification could be discarded in primary care services (without the need to carry out imaging techniques that are usually expensive and invasive) or, alternatively, the patient could be referred to the cardiologist if the test is positive.

For the purpose of the present invention the following terms are defined:
- The expression "reference expression level measured in healthy control subjects" or "reference expression level measured in subclinical aortic-valve calcification control patients", refer to a "reference value" of the expression level of the biomarkers. If a deviation of the expression level (preferably an overexpression) of the biomarkers is determined with respect to said "reference expression level measured in healthy control subjects" or "reference expression level measured in subclinical aortic-valve calcification control patients", this is an indication of CAVD and/or subclinical aortic-valve calcification. Particularly, if the expression level of the biomarkers or signatures of the present invention are significantly higher or lower with respect to said "reference value" this is an indication of CAVD and/or subclinical aortic-valve calcification.
- The expression "risk score" refers to a risk value obtained after processing one or more gene expression values into a single value (or risk value), which represents the probability of disease for the individual. This risk value will be compared with a reference value to evaluate if the patient might be suffering from CAVD and/or subclinical aortic-valve calcification.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skill in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and therefore the threshold value) can be determined using a Receiver Operating Characteristic (ROC) curve based on experimental data. For example, after determining the levels of the biomarkers in a group of reference, one can use an algorithm for the statistic treatment of the measured expression of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0.

- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of". Thus, the phrase "consisting of" indicates that the listed elements are required or mandatory, and that no other elements may be present.

### Brief description of the figures

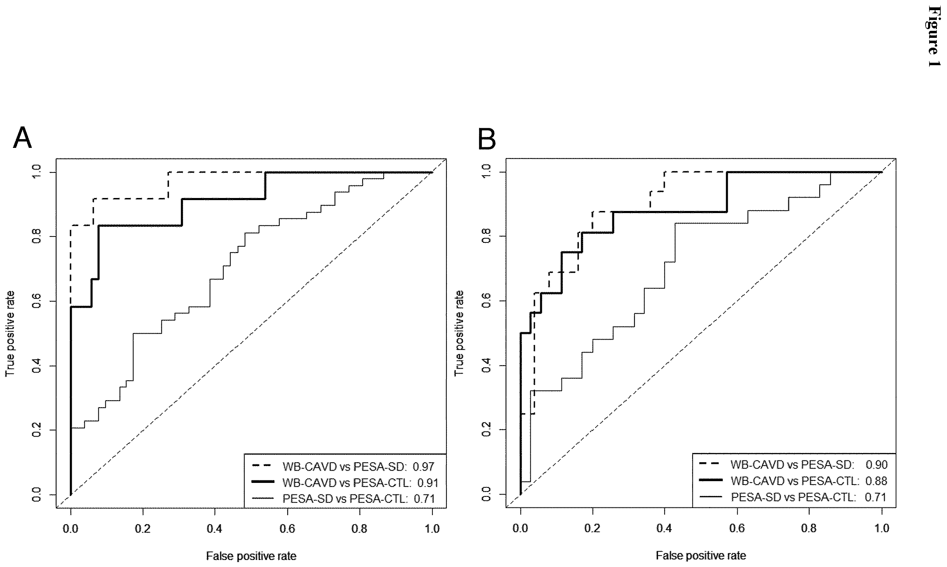

**Figure 1****.** (A) ROC curves. Receiver operating characteristic (ROC) curve of multivariate logistic regression model predicting CAVD (WB-CAVD, n=12) vs individuals without valve disease but with preclinical aortic-valve calcification (PESA-SD, n=52; thick discontinuous line, AUC=0.97) and vs PESA participants without valve calcification (PESA-CTL, n=52; thick solid line, AUC=0.91). A third ROC curve for the prediction of PESA participants with valve calcification (PESA-SD) from those without calcification (PESA-CTL; thin solid line, AUC=0.71) is shown. The thin discontinuous diagonal line indicates a fifty-fifty percent chance to predict each group. **(B)** Follow-up after three years. Validation of the model in a second independent group of CAVD patients (WB-CAVD) vs a subgroup of PESA with preclinical disease obtained three years later (PESA-SD; thick discontinuous line, AUC=0.90) and vs a PESA subgroup without valve calcification (PESA-CTL; thick solid line, AUC=0.88). A third ROC curve for the prediction of PESA participants with valve calcification (PESA-SD) from those without calcification (PESA-CTL; thin solid line, AUC=0.71) is shown. The thin discontinuous diagonal line indicates a fifty-fifty percent chance to predict each group.

### Detailed description of the invention.

### Example 1. Material and methods.

### Example 1.1. RNA-sequencing.

RNA-sequencing was carried out on human foetal aortic valves at gestational weeks 9, 13, and 22, and on a case-control study with adult non-calcified (control) and calcified bicuspid (BAV) and tricuspid (TAV) aortic valves. K-means clustering identified co-expressed gene patterns up- and down-regulated in the different conditions. Canonical pathway analysis revealed a predominant immune-metabolic gene signature indicating ongoing innate and adaptive immune responses, including lymphocyte T cell metabolic adaptation. Cytokine and chemokine signalling, cell migration, and proliferation were all increased in CAVD, whereas oxidative phosphorylation and protein translation were decreased. Discrete immune-metabolic gene signatures were already present at foetal stages and increased in adult controls, suggesting that these processes intensify throughout life and heighten in disease. Cellular stress-response and neurodegeneration gene signatures were aberrantly expressed in CAVD, pointing to a mechanistic link between chronic inflammation and biological ageing. Comparison of the valve RNA-sequencing dataset with a case-control study of whole blood transcriptomes from asymptomatic individuals with early aortic valve calcification identified a highly predictive gene signature of CAVD and of moderate aortic valve calcification in overtly healthy individuals.

### Example 1.2. Study population and aortic valve collection.

*Foetuses.* Human embryos and foetuses (gestational weeks 9 to 13) were obtained from electively terminated pregnancies and were anonymously donated to research after written informed consent from donors in concordance with French legislation (PFS14-011) and with prior approval of the protocol (to S.Z.) from the "Agence de la biomedecine". Human aortic valves from 22-week foetuses were obtained from medically terminated pregnancies after parents had provided their written informed consent to participate in this study, so that this investigation conformed to the principles outlined in the Declaration of Helsinki. Human foetal aortic valves were recognised by their anatomical landmarks under the microscope. Aortic valve leaflets were isolated with minimal aortic wall contamination from a total of 9 foetal hearts, obtained at gestational weeks 9 (n=4), 12 (n=1), 13 (n=2), and 22 (n=2. Samples were immediately flash frozen in liquid nitrogen.

*Patients.* The CAVD study population consisted of patients with BAV (n=5) or TAV (n=7) referred for aortic valve or aortic root surgery at Monteprincipe Hospital and Hospital Clínico San Carlos (Madrid, Spain); patients were prospectively recruited between December 2009 and February 2013. Patients with a history of rheumatic heart disease, infective endocarditis, or connective tissue disorders were excluded. To determine the morphology and functional state of the aortic valve, all patients underwent clinical evaluation and preoperative 2-dimensional echocardiography according to the *American Heart Association* and the *American College of Cardiology* guidelines. The aortic valve morphology and the presence of valvular degeneration, including the extent of leaflet calcification, thickening, prolapse, and/or redundancy, were documented at surgery. Calcification was graded as mild (1/3 leaflet area affected) or severe (2/3 leaflet area affected). Immediately following surgical removal, a portion of the aortic valve leaflet was placed in sterile saline buffer and stored at -80°C until processing. Control valve samples were obtained at autopsy of individuals without cardiac problems who suffered a traumatic death (n=6) or at time of transplantation from heart transplant receptors with normal aortic valves (n=2). All collected samples were used for the study. All patients or close relatives (for autopsy controls) gave written informed consent, and the study was approved by the Health Service Ethics Committees (Comité Ético de Investigación Clínica) of participant centres and the investigation conformed to the principles outlined in the *Declaration of Helsinki.* Informed consent was given prior to their inclusion in the study.

*Whole-blood samples.* Genes of interest were quantified by quantitative reverse transcription-polymerase chain reaction (qRT-PCR) in whole-blood (WB) samples from 104 age-matched case-control male participants in the PESA (Progression of Early Subclinical Atherosclerosis) study. For detailed description of design rationale see ref 12. Cases (PESA-SD) were defined as participants with an aortic-valve calcification score >1, as assessed by the Agatston method *(*Agatston AS, Janowitz WR, Hildner FJ, Zusmer NR, Viamonte M, Jr., Detrano R. Quantification of coronary artery calcium using ultrafast computed tomography. J Am Coll Cardiol 1990;15:827-832). Controls (PESA-CTL) had no calcification of the aortic valves or of the coronary arteries. Whole blood was collected in PAXGene tubes (BDBiosciences) and stored at -80°C before processing.

### Example 1.2. RNA isolation

*Foetuses.* Total RNA from foetal aortic valve leaflets was obtained using the TRIzol LS (Invitrogen, Carlsbad, CA, USA) method and digested with RNase free DNase I (Invitrogen). Isolated RNA was quantified by chip-based capillary electrophoresis (Agilent 2100 Bioanalyzer Pico Chip; Agilent Technologies, Santa Clara, CA, USA).

*Patients.* Total valve RNA was isolated with TRIzol (Invitrogen, Life Technologies). RNA purity, concentration, and integrity were assessed by a combination of Nanodrop 2000 spectrophotometry (Thermo Scientific) and automated electrophoresis in a 2100 Bioanalyzer (RNA6000 Nano LabChip; Agilent). Informed consent was given prior to their inclusion in the study.

*PESA and CAVD patients.* Total RNA was isolated from WB samples in PAXgene blood RNA tubes using either the PAXgene blood mRNA kit (PreAnalytiX, Hombrechtikon, Switzerland) for manual isolation or the QIAsymphony PAXgene blood RNA kit (PreAnalytiX) for automated isolation using a QIAsymphony SP liquid handling robot (Qiagen, Venlo, Netherlands). RNA integrity, purity and concentration were determined using a Nanodrop ND-1000 spectrophotometer (ThermoFisher Scientific, Waltham, MA, USA) and a 2100 Bioanalyzer (Agilent). Only samples with a RIN > 6 were selected for reverse transcription.

### Example 1.3. RNA-seq data generation.

*Foetuses.* RNA-seq libraries were created from RNA samples with an RNA integrity number (RIN) > 7 using the TruSeq Stranded Total RNA with Ribo-Zero Gold Prep Kit (Illumina, San Diego, CA). Final cDNA libraries were checked for quality and quantified in the 2100 Bioanalyzer (Agilent). The libraries were loaded in the flow cell at 8 pM, and clusters were generated in Cbot and sequenced in the Illumina Hiseq 2500 instrument as 50bp single-end reads according to Illumina's instructions. Image analysis and base calling were performed using RTA and CASAVA.

*Patients.* RNA-seq was performed on DNase I-treated RNA samples with a RIN > 7 (Bioanalyzer 2100, Agilent, Santa Clara, CA, USA). Libraries were prepared from 500 ng total RNA using the TruSeq™ RNA Sample Prep Kit v2 (Illumina) and were 75 base single-end sequenced in a Genome Analyzer IIx instrument (Illumina).

### Example 1.4. Bioinformatics analysis of RNA-Seq.

### Data pre-processing

FASTQ files were pre-processed with cutadapt [Martin M. CUTADAPT removes adapter sequences from high-throughput sequencing reads. EMBnetjournal 2011;17:10-12] to eliminate Illumina adapter remains and were aligned to the human reference sequence (GRCh38.78) using RSEM 1.2.31 [Li B, Dewey CN. RSEM: accurate transcript quantification from RNA-Seq data with or without a reference genome. BMC bioinformatics 2011;12:323]*.* Raw data from 27 RNA-seq samples from GSE76718 were downloaded and pre-processed using the same pipeline. Data from the three experiments were normalised with respect to each other using TMM with log-ratio trimming=0.3 and sumTrim=0.05 and voom-transformed. Control samples were present, allowing accurate batch correction of the data using Combat [Johnson WE, Li C, Rabinovic A. Adjusting batch effects in microarray expression data using empirical Bayes methods. Biostatistics 2007;8:118-127*].* Only genes with at least 1 cpm in at least 10 samples were considered for further analysis.

### Differential Expression Analysis

Differential expression between groups was performed using mixed models with eBayes, as implemented in the Biooconductor Limma package *[*Ritchie ME, Phipson B, Wu D, Hu Y, Law CW, Shi W, Smyth GK. limma powers differential expression analyses for RNA-sequencing and microarray studies. Nucleic acids research 2015;43:e47]*.* A gene was classified as differentially expressed if its Benjamini-Hochberg adjusted P-value was < 0.05 in any of the contrasts under study.

### Unsupervised classification

k-means clustering (k=50) was performed using the R ComplexHeatmap package on genes with a |logFC|>=0 in at least one of the comparisons of interest.

### Functional analysis

Ingenuity Pathway Analysis (IPA, Quiagen) was used to recover, analyse, and summarise functional annotations of gene collections in terms of their association with canonical pathways, involvement in biological functions, and dependence on upstream transcriptional regulators. Significance was defined by Benjamini-Hochberg (B-H) adjusted P-value < 0.05.

### Example 1.5. qRT-PCR for validation studies in whole blood.

*Selection of candidate genes for qRT-PCR.* We used whole blood transcriptomics data generated in the PESA study (Fernandez-Ortiz A, Jimenez-Borreguero LJ, Penalvo JL, Ordovas JM, Mocoroa A, Fernandez-Friera L, Laclaustra M, Garcia L, Molina J, Mendiguren JM, Lopez-Melgar B, de Vega VM, Alonso-Farto JC, Guallar E, Sillesen H, Rudd JH, Fayad ZA, Ibanez B, Sanz G, Fuster V. The Progression and Early detection of Subclinical Atherosclerosis (PESA) study: rationale and design. Am Heart J2013;166:990-998*).* In order to identify genes that might potentially serve as predictors of CAVD. The criteria to select these candidate genes were: (i) Genes differentially expressed between individuals with (n=59) and without (n=396) calcification of the aortic valve (p-value<=0.05) and with no evidence to be differentially expressed between individuals with and without calcification in the coronaries (p-value>0.05. (ii) Aortic valve-specific differentially expressed genes with a top 10% correlation between their expression level and the level of calcification of the aortic valves. (iii) Their expression levels in the RNA-Seq samples of the aortic valves were either up-regulated in disease vs no disease samples or they were upregulated in early embryonic valve samples and in the diseased samples but not in the control samples. From the 10 genes fulfilling the three criteria, 6 were selected based on their presumptive function.

*qRT-PCR.* RNA samples from PESA and CAVD patients (75ng) was reverse transcribed using the High Capacity cDNA Reverse Transcription kit with RNase Inhibitor (ThermoFisher Scientific). Gene expression of 6 genes of interest (CD28, ITK, LINS1, ASCC3, PAG1, and NLRC5) and 2 endogenous genes (ACTB and GAPDH) was quantified by qPCR using TaqMan probes (ThermoFisher Scientific) in 52 case-control pairs from the PESA study (case: positive calcification of the aortic valve) and in 12 CAVD patients from Monteprincipe Hospital. Each reaction was performed in triplicate and the final reaction volume was set to 10µl. qPCRs were conducted in 384-well plates (Cat. no. 4344345; Applied Biosystems) using an ABI PRISM 7900HT Sequence Detection System (Applied Biosystems). Each well included 3 µl (2.25 ng) cDNA, 5µl 2x TaqMan Universal Mater Mix (ThermoFisher Scientific), 0.5 µl 20x TaqMan assay (ThermoFisher Scientific), and 1.5 µl H₂O. The cycling protocol was 10 min at 95°C followed by 60 cycles of 15 s at 95°C and 60 s at 60°C. Baseline and threshold were set automatically in SD S 2.3 (ThermoFisher Scientific) to calculate Cq values. The Cq values were then imported into qBasePlus (Biogazelle, Zwijnaarde, Belgium) to calculate relative quantities normalised to GAPDH (not ACTB, due to high variability in the samples of interest). Data were scaled per sample to eliminate potential biases.

### Example 1.6. Statistical analysis.

A linear mixed model was used to assess correlation between expression of each gene in whole blood (WB) and the degree of calcification in the aortic valve for each disease group (PESA-SD, WB-CAVD) separately. Visualization was performed using the multiplot function and the ggplot2 R package. All samples were jointly analysed using a linear mixed model with the origin of the samples as random effect. The same analysis was performed to assess the correlation of the gene expression with the calcification of the coronaries. The four genes which showed no correlation with the amount of calcium in the coronaries (ASCC3, CD28, ITK, LINS1) were selected to build a gene expression signature using a multinomial model with interaction for the prediction of the three groups of individuals with different severity of disease: WB-CAVD, PESA-SD, PESA-CTL. To quantify the predictive power of the gene signature between different groups of individuals a logistic regression model was fit to the data and the corresponding ROC curves were built using the ROCR R package.

### Example 2. Results.

### Example 2.1. Developing valves are transcriptionally more similar to diseased valves than to control valves.

We chose the developmental timepoints based on the availability human foetal valve tissue between weeks 9 and 23, which roughly corresponds to mouse embryonic days (E): 13.5-17.5 [Krishnan et al. Pediatr Res. 2014 December; 76(6): 500-507]. At this developmental stage in mouse, the primitive valve precursors (endocardial cushions) of aortic and pulmonary valves are remodelled by morphogenic processes leading to thinning, elongation, and formation of multi-laminar valve structure. Genome-wide transcriptome profiling was carried out on human aortic valves (gestational weeks 9, n=4; 12-13, n=3 and 22, n=2), as well as on adult control aortic valves and calcified severely stenotic TAV (cTAV) and BAV (cBAV) tissue. To integrate data across studies and generalise our findings, we conducted a meta-analysis with publicly available expression data from adult control valves, cBAVs, and cTAVs [Guauque-Olarte S, Droit A, Tremblay-Marchand J, Gaudreault N, Kalavrouziotis D, Dagenais F, Seidman JG, Body SC, Pibarot P, Mathieu P, Bosse Y. RNA expression profile of calcified bicuspid, tricuspid, and normal human aortic valves by RNA sequencing Physiol Genomics 2016;48:749-761*].* After correcting for batch effects, the first component of the Principal Component Analysis (PCA variance explained=15.24%) separated developing and adult valve samples according to disease severity, ranging from controls (left hand side) to cTAV (right hand side). Interestingly, foetal valves, especially at week 22, clustered with diseased adult valves. In the second component (PC2), early-stage foetal samples (weeks 9 and 13) were separated from the adult controls and disease samples. Differences between datasets did not generally correlate with batch origin, but rather depended on developmental stage and disease state. Moreover, foetal samples appeared to be more closely related to adult diseased samples than to control samples, suggesting that disease pathogenesis can be ascribed, in part, to the repetition of developmental processes.

### Example 2.1. K-means clustering identifies multi-phasic gene expression across developmental and disease states.

Differential expression analysis was initially carried out between each pair of conditions of interest. However, pair-wise comparisons were not adequate to explore dynamic and coordinated gene expression profiles across different developmental and diseased states. Therefore, K-means clustering was performed to identify gene expression patterns common to the different developmental time-points and disease states. Co-expressed genes (ie. genes undergoing similar stage/disease status expression change) likely participate in the same process, and genes that function in the same process are regulated co-ordinatel *[*Niehrs C, Pollet N. Synexpression groups in eukaryotes. Nature 1999;402:483-487*].* K-means analysis computing a fixed set of 50 clusters yielded dynamic expression profiles of simultaneously increasing and decreasing transcripts across the different conditions. There were 6 broad combinations of expression patterns: 1) genes down-regulated in developing valves and up-regulated in control adult valves (e.g. C34); 2) genes down-regulated in control and up-regulated in diseased valves (e.g. C7); 3) genes up-regulated in developing valves, down-regulated in adult control valves, and up-regulated in diseased valves (e.g. C45); 4) genes up-regulated in developing valves and down-regulated in adult control and disease valves (e.g. C37); 5) genes up-regulated in adult control valves and down-regulated in diseased valves (e.g. C16); and 6) genes down-regulated in developing valves, up-regulated in adult control valves, and down-regulated in diseased valves (e.g. C40). There was also multi-phasic transcription over the developmental time-course, yielding multiple pattern combinations. These results reveal a highly dynamic gene expression pattern during valve development that is recapitulated in the disease process.

### Example 2.2. Comparative functional analysis identifies multiple interconnected pathways.

Ingenuity Pathway Analysis (IPA) of biological functions implicated in valve development and calcification identified a plethora of biological processes enriched significantly in 28 gene clusters (P value <0.05). No significantly enriched pathways were detected in the remaining 22 clusters. Unsupervised hierarchical clustering analysis of the significant clusters found that only one (cluster 10) did not overlap with the others in terms of the functional categories enriched. Regrouping the 27 remaining clusters yielded 6 metaclusters (MC), termed MC1-MC6. Hierarchical clustering appeared to be determined by expression patterns at foetal week 13 and in the diseased state (the cBAV and cTAV expression pattern). Foetal week 13 expression tended to be opposite to the expression pattern in the diseased state, with genes highly expressed in adult disease showing low expression at gestational week 13 and vice-versa; however, there were exceptions (clusters 9, 12, 23, and 45). Moreover, there was overlap among metaclusters themselves: genes in clusters 46 and 32 were shared by MC1 and MC2; cluster 12 was shared by MC2, MC3, MC4, and MC6; clusters 12 to 45 were shared by MC2, MC3, and MC4; and clusters 5 and 45 were shared by MC4 and MC6. This result implies that the biological functions in the different MCs are interconnected.

### Example 2.3. Innate and adaptive immune processes are chronically activated in CAVD.

Canonical pathways analysis was performed on all the genes in each MC, independently of the cluster of origin, to summarise biological functions enriched in each MC. Among the transcripts in MC3, this analysis detected enrichment of 168 IPA pathways related to innate-adaptive immune processes, the top 5 pathways were involved in T cell and dendritic cell function. Transcripts in MC3 were mainly involved in innate immunity, including membrane-bound pattern recognition receptors (PRRs) of the Toll-like family of receptors and nucleotide-binding oligomerisation domain-like receptors (NLRs). Other immune effectors included members of the cluster of differentiation (CD) family, interleukins (IL) and their receptors (ILR), chemokines (CXCL) and their receptors (CXCR), integrins (ITG), immunoglobulin heavy chains (IGH), NFKB/REL family members, and multiple components of immune intracellular signalling pathways (IRAK, RHO, SYK, STAT, PLC, PIK3, and PRKC). The remaining 163 enriched biological functions were involved in critical immune-inflammatory cell growth, metabolism and survival processes. Of the nine clusters in MC3, five were related to increased gene expression patterns in all adult samples, both control and CAVD (clusters 22, 9, 34, 36 and 2), whereas four were increased in CAVD only (clusters 49, 44, 7, and 35). The presence of immune-inflammatory gene signatures in control valves would be consistent with the progressive nature of CAVD. Furthermore, discrete gene signatures were present in development at week 9 (clusters 44 and 35), week 13 (cluster 9), and week 22 (clusters 49 and 22), suggesting that immune-inflammatory gene signatures exist at all stages of aortic valve development but involve relatively fewer pathways than those found in healthy or calcified adult valves.

### Example 2.4. Decreased OXPHOS and ribosomal biogenesis in CAVD.

Transcripts in MC2 represented 83 IPA pathways, with the top 5 related to energy metabolism, protein translation, and sirtuin signalling. Transcript changes in MC2 showed strong enrichment in genes for nuclear encoded subunits of the electron transport chain complexes and ribosomal biogenesis. CAVD samples showed relative decreases in numerous mRNAs. These included transcripts encoding NADH:ubiquinone oxidoreductase (NDUF) of Complex I; ubiquinol-cytochrome c reductase (UQCR) of Complex III; cytochrome c oxidase (COX) of Complex IV; ATP synthase (ATP5) of Complex V; mitochondria-related nuclear-encoded transcripts such as translocases of the outer and inner membrane complex (TOM and TIM, respectively; multiple genes encoding ribosomal structural proteins, including 40S and 60S ribosomal subunit proteins (RPS and RPL respectively); and multiple eukaryotic initiation factors (EIF). A similar pattern was observed for the SIRT NAD-dependent acetyltransferase cellular longevity pathway involving SIRT1, SIRT3, and SIRT6 and for genes associated with neurodegenerative disease, like SOD1 and APP. CAVD samples showed increases or decreases for several members of the serine/threonine-protein phosphatase 2A regulatory subunit (PPP2R2), encoded by *PPP2R5E* and *PPP2R2A.* The remaining 78 pathways in MC2 were implicated in protein degradation (Protein Ubiquitination Pathway); cell metabolism (mTOR Signalling); DNA repair (NER Pathway); ATP and NADH production (Glycolysis I); organ size (HIPPO signalling); and multiple stress signalling gene signatures related to AMPK, ER stress (Unfolded protein response), oxidative stress response (NRF2-mediated Oxidative Stress Response), cellular longevity (Telomerase Signalling), and neurodegenerative disease (Huntington's Disease Signalling). Of the nine clusters in MC2, six had, on average, decreased expression in disease conditions (clusters 24, 31, 32, 16, 41, and 1), while one had increased expression in the diseased state (cluster 12) and three were unchanged (clusters 31, 26, and 37). In general, MC2 transcripts had peak expression at week 13, with lower expression at the other developmental stages, suggesting that metabolic processes associated with aortic valve development are dynamically regulated. Overall, this analysis indicates that CAVD is associated with decreased OXPHOS metabolism, altered proteostasis, impaired cellular stress response, and age-related disease.

### Example 2.5. Cell migration and DNA metabolism pathways are re-activated in CAVD.

Transcripts in MC4 and MC6 represented 202 and 23 IPA pathways, respectively. The top five pathways in MC4 were related to cell migration, adhesion, and epithelial cell integrity. There was a strong functional enrichment of transcripts for myosin light chains (MYLs), which showed low expression in development, high expression in adult control valves, and low expression in CAVD. In contrast, genes encoding cardiac muscle alpha actins (ACTs), integrins (ITGs), and roundabout family members (ROBOs) were either re-activated or re-inhibited in CAVD, depending on the transcript.
Other enriched families were ADAMs (a disintegrin and metalloproteases), ADAMTs (a disintegrin and metalloproteinase with thrombospondin motifs), semaphorins (SEMAs), plexins (PLXNs), ephrin receptors (EPHs), actin related protein 2/3 complex subunits (ARPCs), and Rho guanine nucleotide exchange factors (ARHGEFs). Also enriched were key developmental (WNT, PDGF) and chemokine (CXCL12 and CXCR4) signalling pathways. Of the remaining 201 IPA pathways, many were components of fundamental cell and developmental signalling pathways, implicating paxillin, RhoA, IGF-1, ILK, PAK, G α 12/13, FLT3, ErbB, PPAR α /RXR α , glucocorticoid receptor, VEGF, and TGF- *β*, among others.

The top five pathways in MC6 were related to DNA metabolism, including cell cycle control and DNA damage and repair. There was strong functional enrichment of transcript families for E2F transcription factors, connective tissue growth factor (CTGF), cysteine rich protein (Cyr61), nephroblastoma overexpressed gene (nov or CCN), cyclin-dependent kinases (CDKs), RAD proteins, reconstitution of recombinant human replication factor C proteins (RFCs), 14-3-3 phospho-serine/phospho-threonine binding proteins (YWHAs), Fanconi anaemia complementation group proteins (FANCs), Fanconi breast cancer type 1 susceptibility protein (BRCA1), and small ubiquitin-like modifier proteins (SUMOs). Transcripts associated with MC4 and MC6 were highly expressed in development, notably at week 13. Of the 7 clusters associated with these two MCs, 6 were associated with more elevated gene expression in the diseased state (clusters 25, 23, 5, 45, 17, and 21), whereas only one was associated with lower expression (cluster 40). Therefore, early valve development is characterized by high activity of cell adhesion and trans-endothelial migration, cell cycle and DNA repair, and developmental signalling pathways. These developmental processes are silenced in adulthood but become pathologically reactivated during CAVD.

### Example 2.6. Stress response gene signatures in CAVD.

MC1 and MC5 did not overlap significantly with any of the other MCs. Transcripts in MC1 represented 7 pathways related to sphingosine-1-phosphate signalling, dermatan sulfate metabolism, and neurodegenerative disease. There was strong functional enrichment in transcripts for sphingosine-1-phosphate receptor (SPR), γ -secretase complex (PSENEN, NCSTN), calpains (CAPN), mitogen-activated protein kinase (MAPK), and hyaluronidase (HYAL2). Transcripts in MC1 were elevated in adult controls and low in CAVD. MC5 consisted of a single element, the unfolded protein response (UPR) pathway. Transcripts in MC5 were low in controls and marginally elevated in CAVD. CAVD is thus characterised by ER-stress and neurodegenerative disease gene signatures that are also enriched in other MCs, although different genes are involved. For example, ER stress and UPR pathways are also enriched in MC2.

### Example 2.7. Identification of circulating biomarkers predicting CAVD

To identify potential biomarkers useful for predicting CAVD, the full valve dataset was compared with whole blood RNA-Seq data from asymptomatic individuals with aortic valve calcification, detected by non-contrast computed tomography in the *Progression of Early Subclinical Atherosclerosis* (PESA) study *[*Fernandez-Ortiz A, Jimenez-Borreguero LJ, Penalvo JL, Ordovas JM, Mocoroa A, Fernandez-Friera L, Laclaustra M, Garcia L, Molina J, Mendiguren JM, Lopez-Melgar B, de Vega VM, Alonso-Farto JC, Guallar E, Sillesen H, Rudd JH, Fayad ZA, Ibanez B, Sanz G, Fuster V. The Progression and Early detection of Subclinical Atherosclerosis (PESA) study: rationale and design. Am Heart J 2013;166:990-998*].* PESA participants undergo simultaneous cardiac calcium scoring and blood sampling. Out of the 455 PESA participants for which RNA-Seq data was available in visit 2 (three years after recruitment), 59 had evidence of valve calcification and 52 of these had sufficient integrity for qPCR analysis. None of them had valvular stenosis/regurgitation, evidencing an early stage of the disease. Their transcriptome was compared to the transcriptome of PESA individuals without valvular calcification (n=395). From the whole set of genes identified as differentially expressed (p<0.05), six were selected that showed a disease-specific pattern in the RNA-Seq data of human aortic valves. Five of these genes were related to adaptive innate and adaptive responses *(CD28, ITK, PAG1, NLRC5, and ASCC3)* and a sixth was related to the WNT pathway (*LINS1*). The expression of the six genes was measured in whole blood (WB) on 104 PESA participants [52 cases: asymptomatic individuals with calcification of the aortic valve (PESA-SD); 52 controls: individuals without calcification (PESA-CTL)] and 12 patients with CAVD recruited at Monteprincipe Hospital (WB-CAVD) by qRT-PCR and correlated with the degree of aortic valve calcification. Considering all the conditions, all 6 genes individually showed some degree of correlation (*P*≤0.27). Given that, *NLRC5* and *PAG1* were also associated with total calcification of the coronaries they were excluded as potential specific markers of aortic valve calcification.
To determine the utility of the four genes as potential markers of valve calcification, we built a model using the expression measures of the four genes specifically associated with aortic valve calcification: *ASCC3, ITK, CD28* and *LINS1.* A ROC curve was built to represent the predictive value of the model to classify calcific disease patients (WB-CAVD, n=12) vs PESA-SD individuals (n=53; AUC=0.97) and vs PESA-CTL (n=52; AUC=0.91) (**Figure 1A**). An additional model was built to assess the power of our gene signature to distinguish between individuals without calcification (PESA-CTL) from those with subclinical disease (PESA-SD) (AUC=0.71). This model was then validated in whole blood samples of an independent cohort of CAVD patients (n=16), together with samples from 25 of the 52 PESA-SD individuals and 35 of the 52 PESA-CTL individuals taken three years after the visit 2 samples (**Figure 1B**). The predictive power of our model achieved an AUC of 0.90 and 0.88 to discriminate CAVD patients from PESA-SD and PESA-CTL individuals, respectively. The AUC of the model to distinguish PESA-SD from PESA-CTL was again 0.71 (**Figure 1B**). Thus, our model identifies a gene expression signature in blood that is highly predictive of valve disease and of aortic valve calcification levels in otherwise healthy individuals.

## Claims

1. *In vitro* method for the diagnosis and/or prognosis of calcific aortic valve disease (CAVD) or subclinical aortic-valve calcification which comprises: a) Measuring the expression level of a combination of genes comprising at least: [ASCC3, ITK, CD28 and LINS1] in a biological sample obtained from the patient, b) processing the expression level values in order to obtain a risk score and c) wherein if a deviation or variation of the risk score value is identified, as compared with a reference expression level measured in healthy control subjects, this is an indication that the subject is suffering from calcific aortic valve disease (CAVD) or subclinical aortic-valve calcification.

2. *In vitro* method, according to claim 1, for the differential diagnosis between calcific aortic valve disease (CAVD) and subclinical aortic-valve calcification which comprises: a) Measuring the expression level of a combination of genes comprising at least: [ASCC3, ITK, CD28 and LINS1] in a biological sample obtained from the patient, b) processing the expression level values in order to obtain a risk score and c) wherein if a deviation or variation of the risk score value is identified, as compared with the reference expression level measured in subclinical aortic-valve calcification control patients, this is an indication that the subject is suffering from calcific aortic valve disease (CAVD).

3. *In vitro* method for stratifying patients according to their risk of suffering from calcific aortic valve disease (CAVD) or subclinical aortic-valve calcification which comprises: a) Measuring the expression level of a combination of genes comprising at least: [ASCC3, ITK, CD28 and LINS1] in a biological sample obtained from the patient, b) processing the expression level values in order to obtain a risk score and c) wherein if a deviation or variation of the risk score value is identified, as compared with a reference expression level measured in healthy control subjects, this is an indication that the subject is suffering from calcific aortic valve disease (CAVD) or subclinical aortic-valve calcification.

4. *In vitro* method, according to any of the previous claims, wherein the biological sample is selected from: whole blood, serum or plasma.

5. *In vitro* method, according to any of the previous claims, wherein the diagnosis is confirmed by imaging techniques, preferably ultrasound scan.

6. *In vitro* use of a combination of genes comprising at least: [ASCC3, ITK, CD28 and LINS1] for the diagnosis and/or prognosis of calcific aortic valve disease (CAVD) or subclinical aortic-valve calcification.

7. *In vitro* use, according to claim 6, for the differential diagnosis between calcific aortic valve disease (CAVD) and subclinical aortic-valve calcification.

8. *In vitro* use of a combination of genes comprising at least: [ASCC3, ITK, CD28 and LINS1] for stratifying patients according to their risk of suffering from calcific aortic valve disease (CAVD) or subclinical aortic-valve calcification.

9. *In vitro* use, according to any of the claims 6 to 8, in combination with imaging techniques, preferably ultrasound scan.

10. Kit adapted for the diagnosis of calcific aortic valve disease (CAVD), for the diagnosis of subclinical aortic-valve calcification, or for the differential diagnosis between calcific aortic valve disease (CAVD) and subclinical aortic-valve calcification which comprises reagents for the determination of the expression level of a combination of genes comprising at least: [ASCC3, ITK, CD28 and LINS1].

11. Use of the kit of claim 10 for the diagnosis of calcific aortic valve disease (CAVD), for the diagnosis of subclinical aortic-valve calcification, or for the differential diagnosis between calcific aortic valve disease (CAVD) and subclinical aortic-valve calcification.
